# EUROPEAN PATENT SPECIFICATION

(11) **EP 2 323 969 B1**
(45) Date of publication and mention of the grant of the patent: **27.12.2017**
(21) Application number: 09807409.9
(22) Date of filing: 17.08.2009
(51) Int. Cl.: C07C 67/08, C07C 69/533, A61K 8/37, A61Q 1/04, A61Q 1/10, A61G 5/00, A61Q 5/12, A61Q 17/04, A61Q 19/00, A61Q 19/08, A61Q 19/10

(54) **USE OF ESTERS DERIVED FROM NATURAL SOURCES HAVING LOWER VISCOSITY AND HIGHER SPREAD RATE AS WELL AS PREPARATION OF NATURAL PERSONAL CARE COMPOSITION COMPRISING SUCH ESTERS**
ANWENDUNG VON ESTERN AUS NATÜRLICHEN QUELLEN MIT GERINGERER VISKOSITÄT UND HÖHERER VERTEILUNGSRATE, und HERSTELLUNG VON ENTSPRECHENDEN NATÜRLICHEN KÖRPERPFLEGEZUSAMMENSETZUNGEN
UTILISATION D´ESTERS DÉRIVÉS DE SOURCES NATURELLES AYANT UNE VISCOSITÉ RÉDUITE ET UNE VITESSE DE PROPAGATION ACCRUE et PRÈPARATION DE COMPOSITIONS NATURELLES DE SOINS PERSONNELS LES COMPRENANT

(30) Priority: 15.08.2008 US 89347 P
(43) Date of publication of application: 25.05.2011
(60) Divisional application: 17202262.6
(73) Proprietor: Inolex Investment Corporation, Wilmington, DE 19810 (US)
(72) Inventor: BURGO, Rocco, Mullica Hill NJ 08062 (US); PARKER, Jeffrey, New Hope PA 18938 (US); WINN, Daniel, Kingston NJ 08528 (US)
(74) Representative: Petraz, Gilberto Luigi
(86) International application number: PCT/US2009/054006
(87) International publication number: WO 2010/019939

(56) References cited:
- US-A- 2 585 053
- US-A- 5 686 087
- US-A- 5 693 255
- US-B1- 6 495 097

## Description

### BACKGROUND OF THE INVENTION

As the population becomes aware of the potential adverse effects to the body and to the environment associated with use of ingredients derived from fossil fuels, the personal care industry has advanced its search for "natural" ingredients. Although the term "natural" currently has no industry standard definition, efforts are under way by industry trade organizations to devise a more uniform, concise meaning. Currently, it is generally recognized that materials derived from renewable and/or sustainable or otherwise non-fossil fuel sources, are considered to be natural. Personal care compositions containing these materials may be marketed accordingly. Currently, it is the industry consensus that petrochemicals and petrochemically-derived materials are not "natural". There is also a trend within the trade to eliminate animal-derived materials and plant-derived materials that are obtained from the use of genetically modified organisms. Compounds made by certain chemical processes may also be considered unsuitable for use in "natural" personal care compositions.

In skin care, the tactile impression or skinfeel of a product, such as the feeling upon initial application (initial feel), the feeling during spreading of the product over the skin (rub-out), and the feeling after application is complete (after feel), contributes to the product's commercial success or failure. Other characteristics, such as fragrance and taste or lack thereof, may be equally important. Generally, emollients are the components of a composition that are directly related to the "feel" or tactile impression properties, because they provide lubrication, humectancy, and occlusion.

The spreading rate and viscosity are properties of an emollient that contribute to skinfeel or tactile impression. Rapidly spreading/low viscosity products are perceived as "light", whereas slow spreading/higher viscosity products are perceived as "heavy." While heavy feeling products are sometimes preferred, for example, in skin care products such as massage oils, ointments, and barrier creams, lightness as well as a lack of oiliness and/or greasiness is preferred in many applications. Other terms used to describe lightness of a personal care composition may be "dry," "velvety," and "silky." Additionally, in most instances, it is preferable that the emollient(s) is substantially and/or completely free of color and/or taste. It is also desired that they be non-flammable and toxicologically benign.

Vegetable oil emollients have been used in personal care formulations for centuries. Vegetable oils are natural; thus, a personal care formulator preparing a "natural" product may choose a vegetable oil over a petrochemically-derived synthetic, even when the magnitude of benefits realized is lesser as compared to that which would be obtained by use of the petrochemically-derived alternative. Common vegetable oils used in personal care compositions include coconut oil, corn oil, cottonseed oil, canola oil, olive oil, palm oil, peanut oil, safflower oil, sesame oil, soybean oil, sunflower oil and jojoba oil. The viscosities of exemplary vegetable oils are listed in Table 1, and, as can be seen, are relatively high.

**TABLE 1**

| Viscosity of Common Vegetable Oils | |
|---|---|
| Common name | Viscosity, centistokes at 25°C |
| Corn Oil | 65 |
| Canola Oil | 67 |
| Olive Oil | 87 |
| Soybean Oil | 69 |
| Jojoba Oil | 130 |

Low viscosity emollients that can deliver a light feeling to the skin can be made using entirely non-petrochemically-derived starting materials; however, the availability of suitable starting materials is limited. For example, monoesters of low viscosity and light skinfeel can be synthesized by esterifying ethanol from fermentation of corn, sugar cane, beets, and/or other plants with fractionated vegetable fatty acids of lower chain length, normally derived from coconut or palm kernel oil. Although this method can provide low viscosity, the monoesters derived therefrom have a low molecular weight and tend to be volatile, and therefore odorous, which makes their use limited.

Alternatively, "natural" esters for use in personal care compositions may be derived from the esterification of glycerol from vegetable sources with fractionated vegetable derived fatty acids of lower chain length. The most common of these is glyceryl tricaprate/tricaprylate (Lexol GT 8/65, Inolex Chemical Company, Philadelphia, PA) in which the capric and caprylic acid is obtained from splitting and fractionation of coconut or palm kernel oil. The glyceryl tricaprate/tricaprylate material has a viscosity of approximately 25-30 centistokes (at 25°C) and a spreading value of 7.79 (5 minutes, cm²) and has been characterized as having a light skinfeel.

However, the principle remains that odorless, lower viscosity synthetic esters having higher spreading values are considered preferable when one wishes to devise a formulation providing a light skinfeel. For this reason, often petrochemically-derived materials fitting this definition are selected for use over any glyceryl tricaprate/tricaprylate material or other natural materials, since these natural materials do not provide the same performance benefits. Examples of such petrochemically-derived synthetic esters widely used within the personal care industry are neopentyl glycol diheptanoate (LexFeel 7, Inolex Chemical Company, Philadelphia, PA, USA) and isononyl isononanoate (Dermol 99, Alzo International, Sayreville, NJ, USA.) U.S. 4,322,545, 4,323,693, and 4,323,694 to Scala, and U.S. 6,365,629 to Zofchak et. al, incorporated herein by reference, also describe wholly or partially petrochemically-derived esters useful in personal care formulations for providing "dry emolliency" and/or "light feeling" to the formulation. Furthermore, document US 2,585,053 discloses solid esters for compounding ingredients of rubbers, other elastomers and plastics and document US 6,495,097 discloses fragrance and flavor compositions containing odor neutralizing agents. Moreover, document US 5,686,087 discloses cosmetic and/or pharmaceutical formulations with an improved feeling on the skin based on mixed guerbet alcohols.

Accordingly, there is a need in the art for esters that can provide a light skinfeel when incorporated as an emollient into personal care formulations and which meet the current "natural" standard. Ideally, such materials would have viscosities and spreading values similar to the petrochemical alternatives. Furthermore, the esters would preferably be low odor, low taste, non-flammable and toxicologically benign.

### BRIEF SUMMARY OF THE INVENTION

The invention includes the use of a natural ester for topical application to the skin, hair, and/or nails comprising the esterification product of (1) an undecylenic acid derived from a natural source and (2) a vegetable-derived fatty alcohol chosen from the group consisting of hexyl alcohol, heptyl alcohol, caprylyl alcohol, nonyl alcohol, decyl alcohol, and undecyl alcohol and combinations thereof, wherein the ester provides a perception of a light skinfeel to a user. Also disclosed are methods of preparing a natural personal care composition comprising combining an ester and at least one personal care component, wherein the ester comprises the esterification product of (1) an undecylenic acid derived from a natural source and (2) a vegetable-derived fatty alcohol chosen from the group consisting of hexyl alcohol, heptyl alcohol, caprylyl alcohol, nonyl alcohol, decyl alcohol, and undecyl alcohol and combinations thereof, wherein the ester provides a perception of a light skinfeel to a user. Methods of altering the tactile impression and/or skinfeel provided to a user by a personal care composition are also disclosed.

### DETAILED DESCRIPTION OF THE INVENTION

The esters disclosed therein are natural and can be used to prepare natural personal care compositions. By "natural" it is meant that the starting acids and alcohols are wholly derived from renewable and/or sustainable sources, and are not derived from fossil fuels or any other petrochemical sources.

The natural ester includes the esterification product of at least one undecylenic acid that is derived from a natural source. By "natural source", it is meant that the starting acid is derived from a renewable and/or a sustainable resource, and not derived from fossil fuel or any other petrochemical sources. For example, the starting acid may be derived from a vegetable oil, such as those obtained from oil-bearing seeds or other botanical source. Exemplary oils include almond oil, castor oil, coconut oil, corn (maize) oil, cottonseed oil, canola oil, flax seed oil, hempseed oil, nut oil(s), olive oil, palm oil, peanut oil, safflower oil, sesame oil, soybean oil, sunflower oil, jojoba oil and combinations of these oils. In an embodiment, preferably the starting acid is derived from castor oil.

The ester disclosed therein is prepared by esterifying the acid described above with a vegetable-derived fatty alcohol. The fatty alcohol can be derived from any plant or vegetable source, including the oils listed above. Suitable vegetable-derived fatty alcohols include, hexyl alcohol, heptyl alcohol, caprylyl alcohol, nonyl alcohol, decyl alcohol, and undecyl alcohol. Additionally, the ester may be prepared using a mixture or combination of one or more vegetable-derived fatty alcohols.

In an embodiment, the ester is prepared from a vegetable-derived fatty alcohol that is heptyl alcohol and the resultant esterification product is heptyl undecylenate. In an embodiment, the ester is a compound represented by the Formula (I).

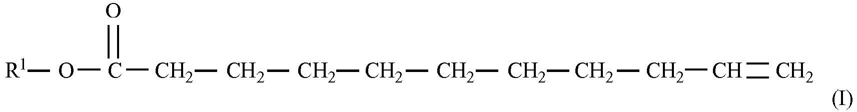

In formula (I), R¹ is an alkyl group. The alkyl group may contain three to twenty-five carbon atoms. However, it may be preferred that the alkyl group of R¹ is a group having six to twelve carbon atoms. Alternatively, it may be preferable that R¹ is chosen from an alkyl group having seven, eight, nine, ten, or eleven carbon atoms. In the formula (I), any of the carbon atoms may be independently substituted or unsubstituted. Suitable substituent groups may include any and all known in the art, such as, for example, methyl groups, butyl groups, ethyl groups, alkoxy groups, branched and/or unbranched alkyl groups, alkyenyl groups, and halogen atoms.

In an embodiment, it may be preferred that R¹ is derived from a vegetable-derived fatty alcohol, such as, for example, hexyl alcohol, heptyl alcohol, caprylyl alcohol, nonyl alcohol, decyl alcohol, and undecyl alcohol.

The selected acid(s) and vegetable-derived fatty alcohol(s) may be esterified by any means known or developed in the art. For example, esterification may be accomplished by application of heat in the presence of a catalyst, or in the absence of a catalyst, using an excess of the more volatile alcohol component to drive the reaction to high conversion. Neutralization, distillation, adsorption, and filtration processes may be used to purify the product. It may be preferred that the method employed is one that leads to an ester of high purity and low color and odor. Such reactions are well within the skill of a person of ordinary skill in the art and are routinely and conventionally executed in the industry.

The ester described herein preferably has a lower viscosity, for example, about 2 to about 10 centistokes at 25°C, alternatively about 3 to about 7 centistokes at 25°C. Preferably, the spreading values of the ester indicate a rapidly spreading material. For example, the spread values may be about 6 to about 8 (5 minute, cm²). These properties contribute to the skinfeel properties/tactile impression of the esters and/or personal care compositions in which it is included; specifically, users perceive a light, silky, and/or dry skinfeel upon application.

The invention also discloses personal care compositions that include the ester. Personal care compositions may include, oral care products, skin cleansing products, hair cleansing products, nail preparations (nail polish, nail polish removers, cuticle and/or nail treatments), conditioning agents for skin, nails and hair, antiperspirants and deodorants, soaps, hair sprays, gels, hair shampoo, hair conditioner, pomades, powders, cosmetics, compositions that are subsequently impregnated into textiles for cleansing or other purposes, lipstick, lotions for skin and hair, including those containing sunscreens or UV absorbers, bath or shower oils, lip gloss, lipsticks, cosmetics for use in pigmenting the eye area, such as eyebrow pencils or powders, eye shadows, and eye liners, hand lotions and salves, creams and lotions for the facial area, hair creams, mousses, gels, and other styling aids, mascara, foundations for application to the face or tanning products.

There personal care composition may contain any additional additives or components useful in formulating a product with the desired end benefits. In an embodiment, it may be desirable to include one or more vegetable oils in the product, such as, for example, almond oil, castor oil, coconut oil, corn (maize) oil, cottonseed oil, canola oil, flax seed oil, hempseed oil, nut oil, olive oil, palm oil, peanut oil, safflower oil, sesame oil, soybean oil, sunflower oil, jojoba oil and combinations of these oils.

Surfactants may be included in the personal care composition, such as, for example, an anionic surfactant, a zwitterionic surfactant, a cationic surfactant, a non-ionic surfactant and combinations of these.

Other exemplary components may include, lipids, alcohols, waxes, pigments, vitamins, fragrances, bleaching agents, antibacterial agents, anti-inflammatory agents, antimycotic agents, thickeners, gums, starches, chitosan, polymeric materials, cellulosic materials, glycerin, proteins, amino acids, keratin fibers, fatty acids, siloxanes, botanical extracts, abrasives and/or exfoliants (chemical or mechanical), anticaking agents, antioxidant agents, binders, biological additives, buffering agents, bulking agents, chelating agents, chemical additives, denaturants, external analgesics, film formers, humectants, opacifying agents, pH adjusters, preservatives, propellants, reducing agents, sunscreen agents, skin darkening agents, essential oils, skin sensates, and combinations of these.

Also included in the invention is a method of preparing a natural personal care composition used for topical application to the skin, hair and/or nails characterized in that it comprises combining an ester and at least one component of a personal care composition, wherein the ester comprises the esterification product of (1)an undecylenic acid derived from a natural source and (2) a vegetable-derived fatty alcohol chosen from the group consisting of hexyl alcohol, heptyl alcohol, caprylyl alcohol, nonyl alcohol, decyl alcohol and undecyl alcohol and combinations thereof, wherein the ester provides a perception of a light skinfeel to the user. Such component may include any known in the art, such as, for example, those listed above. In an embodiment, the preferred component is a vegetable oil or an essential oil. In an embodiment of the invention, methods of altering or modifying the tactile impression and/or skinfeel provided to a user by application of an emollient personal care composition are disclosed. Such method includes incorporating an ester and at least one component that is chosen from a conventional personal care ingredient component, such as, for example, a gum, a natural oil, a cellulosic compound, a salt, a wax, and/or any of the other components listed above.

All ASTMs referred to below are attached as Exhibit A; their contents are incorporated into this text by reference.

### EXAMPLE 1

The physical properties of nine esters of the invention are evaluated and compared to two petrochemically-derived esters and one "natural" ester that are currently used in the art.

Kinematic viscosity was tested at 25°C using ASTM (American Society of Testing and Materials, West Conshohocken, Pennsylvania, USA) official method number D-445.

Color was measured using ASTM D-1209, and flash point was determined using ASTM D-92.

Odor was evaluated olfactorily by a human subject.

To test the spreading characteristics, a 110mm Whatman #4 filter paper was positioned horizontally over an open 8 ounce jar. Fifty microliters of each product were then pipetted onto the center of the filter paper. The spreading area of the liquid was then measured at intervals of one, three, and five minutes. More rapidly spreading products will have a higher spreading area at each time interval.

Table 2 shows the properties of the esters of the invention in comparison to glyceryl tricaprate/tricaprylate material and two preferred petrochemically-derived emollients.

**TABLE 2**

| SAMPLE | A | B | C | D | E | F | G | H | I |
|---|---|---|---|---|---|---|---|---|---|
| Derivation | Natural | Natural | Natural | Natural | Natural | Natural | Natural | Part Petro. | Fully Petro. |
| Physical Form at R.T. | Liquid | Liquid | Liquid | Liquid | Liquid | Liquid | Liquid | Liquid | Liquid |
| Viscosity at 25°C, cSt | 2.42 | 5.67 | 6.53 | 7.38 | 8.3 | 9.3 | 27.2 | 7.2 | 12.0 |
| Viscosity at 40°C, cSt | 1.73 | 4.0 | 4.57 | 5.05 | 5.7 | 6.2 | 21.1 | 5.6 | N/A |
| Spreading Area (1 minute,) cm² | 7.79 | 7.38 | 7.14 | 6.99 | 6.83 | 6.23 | 4.40 | 6.38 | 7.07 |
| Spreading Area (3 minutes,) cm² | 9.99 | 9.7 | 9.34 | 8.99 | 8.72 | 8.29 | 6.68 | 8.38 | 9.34 |
| Spreading Area (5 minutes,) cm² | 11.44 | 11.24 | 10.55 | 10.36 | 10.08 | 9.25 | 7.79 | 9.89 | 10.26 |
| Odor | Very Mild | Very Mild | Very Mild | Very Mild | Very Mild | Very Mild | Very Mild | Very Mild | Very Mild |
| Color (APHA) | 14 | 5 | 8 | 6 | 6 | 11 | 20 | 17 | 17 |
| Flash Point, °C | 174 | 178 | 180 | 198 | 194 | 180 | 241 | 175 | 175 |

Samples were as follows: A = hexyl undecylenate; B = heptyl undecylenate; C = caprylyl undecylenate; D = nonyl undecylenate, E = decyl undecylenate; F = undecyl undecylenate; G = glyceryl tricaprate/tricaprylate; H = neopentyl glycol diheptanoate; and
I = isononyl isononanote.

The results show that the natural esters of the invention possess spreading properties substantially equal to or superior to the petrochemically-derived alternatives.

### EXAMPLE 2

### SPF 15 Skin Lotion

The following example illustrates the use of the invention to prepare a SPF 15 lotion in which the petrochemically-derived neopentyl glycol diheptanoate, an ingredient necessary to provide a light skinfeel in the comparative formulation, is replaced by the natural heptyl undecylenate in the inventive formulation, resulting in a light feeling natural SPF 15 lotion that is entirely free of petrochemically-derived ingredients.

| | INGREDIENT | COMPARATIVE PETROCHEMICAL %, WEIGHT | INVENTIVE NATURAL %, WEIGHT |
|---|---|---|---|
| Phase A | Water | 64.9 | 64.9 |
| | Glycerin | 5.00 | 5.00 |
| | Xanthan Gum | 0.50 | 0.50 |
| | Glucose | 2.00 | 2.00 |
| | Sodium Borate | 0.30 | 0.30 |
| | Sucrose Stearate | 1.50 | 1.50 |
| Phase B | Hempseed Oil | 4.00 | 4.00 |
| | Sucrose Distearate | 1.50 | 1.50 |
| | Nepentyl Glycol Diheptanoate | 7.00 | --- |
| | Heptyl Undecylenate | --- | 7.00 |
| | Beeswax | 5.00 | 5.00 |
| | Aluminum hydroxide | 0.10 | 0.10 |
| | Silica | 0.50 | 0.50 |
| | Titanium Dioxide | 7.00 | 7.00 |
| Phase C | Sandalwood Oil | 0.20 | 0.20 |
| Phase D | Natural Preservative | 0.50 | 0.50 |
| | | 13.3.00 | 100.00 |

The formulation was prepared by combining (in separate vessels) the ingredients of each phase A and phase B and heating each the phase A materials and the phase B materials to 75°C while propeller mixing. Next, the phase A materials and the phase B materials were combined together and homogenized for five minutes. The material was cooled to 45°C while sweep mixing and phase C was added. Cooling was allowed to progress under sweep mixing until the material reached a temperature of 35°C. Phase D was added and mixing continued until the material reached a temperature of 30°C. Mixing was discontinued.

Upon application to the skin, users reported a perception of a light skinfeel.

### EXAMPLE 3

### Natural Tinted Facial Moisturizer

Often, it is desired that creams and lotions that are applied to the face be light in feeling upon initial application, but also provide higher order moisturization benefits that are typically conferred by higher molecular weight, occlusive materials. In this example, the vegetable oils sunflower and coconut are included to provide moisturization, while the ester of the invention, heptyl undecylenate provides perception to the user of an initial light feeling. In the absence of the ester of the invention, this formula is perceived to be heavy, oily and greasy, whereas this formulation is perceived by users to be light because of the "initial feel" contributed by the ester of the invention.

| | INGREDIENT | %, WEIGHT |
|---|---|---|
| Phase A | Water | 67.19 |
| | | |
| | Glycerin | 5.00 |
| | Xanthan Gum | 0.50 |
| | Glucose | 1.00 |
| | Sodium Borate | 0.30 |
| | Sucrose Stearate | 1.50 |
| Phase B | Sunflower Oil | 4.00 |
| | Coconut Oil | 5.00 |
| | Heptyl Undecylenate | 7.00 |
| | Beeswax | 5.00 |
| | Iron Oxide Black | 0.01 |
| | Iron Oxide Yellow | 0.30 |
| | Iron Oxide Red | 0.70 |
| | Titanium Dioxide | 2.00 |
| Phase C | Natural Preservative | 0.50 |
| | | 100.00 |

The facial moisturizer was prepared using the process of Example 1. Upon application to the facial skin surface, users reported a light skinfeel and sufficient moisturization.

### EXAMPLE 4

### Natural Lipgloss

| INGREDIENT | %, WEIGHT |
|---|---|
| Grapeseed Oil | 29.25 |
| Beeswax | 40.00 |
| Coconut Oil | 10.00 |
| Lanolin | 5.00 |
| Coco butter | 5.00 |
| Tocopheryl Acetate | 0.50 |
| Heptyl Undecylenate | 10.00 |
| Flavor | 0.25 |
| | 100.00 |

The lipgloss was prepared by combining the above ingredients and heating to a temperature of 75°C while propeller mixing until a uniform mixture was obtained. The gloss was cooled to 70°C and packaged.

### EXAMPLE 5

### Natural Bath Oil

| INGREDIENT | %, WEIGHT |
|---|---|
| Sweet Almond Oil | 63.5 |
| Heptyl Undecylenate | 35 |
| Tocopheryl Acetate | 1.0 |
| Fragrance | 0.5 |
| | 100.00 |

The ingredients were combined until uniformly mixed and packaged.

### EXAMPLE 6

### Natural Hair Conditioner

| | INGREDIENT | %, WEIGHT |
|---|---|---|
| Phase A | Water | 74.20 |
| | Glycerin | 5.00 |
| | Honey | 0.50 |
| | Glucose | 2.00 |
| | Betaine | 0.30 |
| | Citric Acid | 0.50 |
| Phase B | Brassicamidopropyl Dimethylamine | 2.00 |
| | Cetearyl Alcohol | 10.00 |
| | Heptyl Undecylenate | 5.00 |
| Phase C | Preservative | 0.50 |
| | | 100.00 |

The hair conditioner was prepared using the procedure described in Example 1.

### EXAMPLE 7

### Natural Eye Liner/Brow Pencil

| INGREDIENT | %, WEIGHT |
|---|---|
| Sunflower Oil | 36 |
| Heptyl Undecylenate | 25 |
| Japan Wax | 12.00 |
| Carnauba Wax | 10.00 |
| Zinc Oxide | 4.00 |
| Beeswax | 2.00 |
| Tocopheryl Acetate | 1.00 |
| Mica and Titanium dioxide and Iron Oxide | 10.00 |
| | 100.00 |

The eye liner/brow pencil was prepared by combining the ingredients and heating them to a temperature of 75°C while propeller mixing. Once uniform mixing was achieved, the mixture was cooled to 70°C and packaged.

### EXAMPLE 8

### Natural Eye Shadow

| INGREDIENT | %, WEIGHT |
|---|---|
| Sunflower Oil | 38.00 |
| Heptyl Undecylenate | 25.00 |
| Beeswax | 12.00 |
| Carnauba Wax | 4.00 |
| Zinc Oxide | 8.00 |
| Tocopheryl Acetate | 1.00 |
| Canella wax | 2.00 |
| Mica and Titanium dioxide and Iron Oxide | 10.00 |
| | 100.00 |

The eye shadow was prepared using the procedure described in Example 7 and packaged.

### EXAMPLE 9

| | Natural Hand Salve |
|---|---|
| INGREDIENT | %, WEIGHT |
| Sweet Almond Oil | 41.80 |
| Heptyl Undecylenate | 32.00 |
| Beeswax | 25.00 |
| Comfrey Root Extract | 1.00 |
| Rosemary Oil | 0.10 |
| Lavender Oil | 0.05 |
| Eucalyptus Oil | 0.05 |
| | 100.00 |

The hand salve was prepared using the procedure described in Example 7 and packaged.

### EXAMPLE 10

### Natural Carrot Day Cream

| | INGREDIENT | %, WEIGHT |
|---|---|---|
| Phase A | Water | 67.60 |
| | Glycerin | 4.00 |
| | Xanthan Gum | 0.40 |
| | Glucose | 1.00 |
| | Sodium Borate | 0.30 |
| Phase B | Wheat germ Oil | 1.50 |
| | Sunflower Oil | 3.00 |
| | Coconut Oil | 4.00 |
| | Heptyl Undecylenate | 5.00 |
| | Beeswax | 5.00 |
| | Tocopheryl Acetate | 0.50 |
| | Sucrose Stearate | 2.00 |
| | Stearic Acid | 4.00 |
| | Orange wax | 1.00 |
| Phase C | Beta-carotene | 0.20 |
| Phase D | Preservative | 0.50 |
| | | 100.00 |

The carrot day cream was prepared using the procedure described in Example 1.

### EXAMPLE 11

### SPF 30 Cream

| | INGREDIENT | %, WEIGHT |
|---|---|---|
| Phase A | Water | 25.50 |
| | Avondale Angustifolia (Lavender) Hydroflorate | 30.00 |
| | Carbomer | 0.20 |
| | Sodium Benzoate | 0.20 |
| | Potassium Sorbate | 0.30 |
| Phase B | Octinoxate | 7.00 |
| | Oxybenzone | 5.00 |
| | Octisalate | 4.50 |
| | Octocrylene | 5.50 |
| | Titanium Dioxide | 3.50 |
| | Zinc Oxide | 0.50 |
| | Heptyl Undecylenate | 5.00 |
| | Helianthus Annus (Sunflower) Seed Oil | 0.50 |
| | Stearyl Alcohol | 4.00 |
| | Glyceryl Stearate | 1.50 |
| | Steareth 20 | 3.00 |
| | Prunus Amygdalus Dulcis Oil | 0.50 |
| | Camellia Kissi Seed Oil | 0.50 |
| | Steareth 2 | 1.50 |
| Phase C | Camellia Sinensis (Green Tea) Leaf Extract | 0.10 |
| | Aloe Barbadensis (Aloe Vera) Leaf Juice | 0.10 |
| | Deep Sea Algae Extract | 0.10 |
| | Symphytum Officinale (Comfrey) Leaf Extract | 0.10 |
| | Calendula Officinalis (Marigold) Flower Extract | 0.10 |
| | Citrus Grandis (grapefruit) Fruit Extract | 0.50 |
| Phase D | Sodium Hydroxide | 0.10 |
| Phase E | Essential Oil Blend | 0.20 |
| | Total | 100.00 |

This cream was prepared using the procedure of Example 1.

### EXAMPLE 12

### Natural Anti-Age Serum

| INGREDIENT | %, WEIGHT |
|---|---|
| Water | 92.60 |
| Corn Starch | 2.00 |
| Lecithin | 1.00 |
| Polysorbate 20 | 1.00 |
| Heptyl Undecylenate | 1.50 |
| Calcium Ascorbate | 0.05 |
| Zinc Ascorbate | 0.05 |
| Ascorbal Phosphate | 0.05 |
| Ascorbic acid | 0.10 |
| Magnesium Ascorbal Palmitate | 0.20 |
| Dextrin Palmitate | 0.15 |
| Camellia Sinensis (Green Tea) Leaf Extract | 0.20 |
| Citrus Grandis (grapefruit) Fruit Extract | 0.20 |
| Fragrance | 0.10 |
| Preservative | 0.80 |
| | 100.00 |

The ingredients for the serum were combined with propeller mixing until uniform, then the serum was packaged.

### EXAMPLE 13

### Hair Texturizing Cream

| | INGREDIENT | %, WEIGHT |
|---|---|---|
| Phase A | Water | 88.67 |
| | Glycerin | 2.00 |
| | Aloe Barbadensis Gel (Aloe Vera | 0.10 |
| | Aminomethyl Propanediol | 0.10 |
| | Carbomer | 0.30 |
| | Benzoic Acid | 0.05 |
| | Phenoxyethanol, | 0.80 |
| | Glycereth 2 Cocoate | 0.20 |
| | Sodium Benzoate | 0.20 |
| Phase B | Stearic Acid | 4.00 |
| | Heptyl Undecylenate | 2.00 |
| | Cetyl Alcohol | 1.00 |
| | Limanthes Alba Seed Oil (Meadowfoam), | 0.50 |
| Phase C | Citrus Medica Limonium Peel Oil (Lemon) | 0.02 |
| | Citrus Aurantum Dulcis Oil (Orange | 0.02 |
| | Citrus Grandis Peel Oil (Grapefruit) | 0.02 |
| | Citrus Aurantium Bergamia Fruit Oil (Bergamot) | 0.02 |
| | | 100.00 |

This cream was prepared using the procedure of Example 1.

### EXAMPLE 14

### Natural Moisturizing Night Cream

| | INGREDIENT | %, WEIGHT |
|---|---|---|
| Phase A | Water | 65.59 |
| | Glycerin | 3.00 |
| | Aspalathus Linearis Leaf Extract (red tea | 0.10 |
| | Copper Gluconate | 0.02 |
| | Zinc Gluconate | 0.02 |
| | Manganese Gluconate | 0.02 |
| | Aloe Barbadensis Leaf Gel | 0.10 |
| | Carbomer | 0.20 |
| | Sodium Benzoate | 0.20 |
| | Sodium PCA | 0.20 |
| | Allantoin | 0.10 |
| | Potassium Cetyl Phosphate | 0.50 |
| | Potassium Sorbate | 0.20 |
| Phase B | Stearic Acid | 3.00 |
| | Heptyl Undecylenate | 10.00 |
| | Stearyl Alcohol | 2.00 |
| | Polysorbate 60 | 1.30 |
| | Simmondsia Chinensis Seed Oil | 2.00 |
| | Prunus Amygdalus Dulcis Oil | 3.00 |
| | Glyceryl Laurate | 1.50 |
| | Cetearyl Alcohol | 3.00 |
| | Ceteareth 20 | 2.00 |
| | Sorbitan Stearate | 1.00 |
| | Tocopheryl Acetate | 0.10 |
| Phase C | Sodium Ascorbyl Phosphate | 0.50 |
| Phase D | Benzyl Alcohol | 0.3 |
| Phase E | Potassium Hydroxide | 0.05 |
| | Total | 100.00 |

This cream was prepared using the procedure of Example 1.

### EXAMPLE 15

### Light Sesame Oil Based Mixture

The following example illustrates how the esters described herein may be admixed with a vegetable oil. Admixtures of this type may be preferred by cosmetic formulators. The following ingredients are combined together in the proportion show below:

| INGREDIENT | %, WEIGHT |
|---|---|
| Sesame Oil | 80.0 |
| Heptyl Undecyleneate | 20.0 |

## Claims

1. Use of a natural ester for topical application to the skin, hair, and/or nails **characterized in that** it comprises the esterification product of (1) an undecylenic acid derived from a natural source and (2) a vegetable-derived fatty alcohol chosen from the group consisting of hexyl alcohol, heptyl alcohol, caprylyl alcohol, nonyl alcohol, decyl alcohol, and undecyl alcohol and combinations thereof, wherein the ester provides a perception of a light skinfeel to a user.

2. The use of claim 1, wherein the undecylenic acid is derived from castor oil.

3. The use of claim 3, wherein the vegetable-derived fatty alcohol is heptyl alcohol.

4. The use of claim 1, wherein the esterification product is heptyl undecylenate.

5. The use of claim 1, wherein said esterification product is an ester represented by the Formula (I): 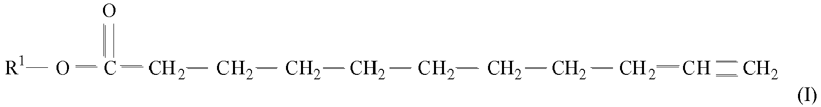 wherein R¹ is chosen from an alkyl group.

6. The use of claim 5, wherein R¹ is chosen from an alkyl group having six to twelve carbon atoms.

7. The use of claim 5, wherein R¹ is chosen from an alkyl group having seven, eight, nine, ten, or eleven carbon atoms.

8. The use of claim 5, wherein one or more carbon atom(s) is independently substituted.

9. The use of claim 1, wherein the viscosity of the fluid is 2 to 10 centistokes at 25°C.

10. The use of claim 1, wherein the spreading value of the fluid is 6 to 8 (1 minute, cm²).

11. A method of preparing a natural personal care composition used for topical application to the skin, hair, and/or nails **characterized in that** it comprises combining an ester and at least one component of a personal care composition, wherein the ester comprises the esterification product of (1) an undecylenic acid derived from a natural source and (2) a vegetable-derived fatty alcohol chosen from the group consisting of hexyl alcohol, heptyl alcohol, caprylyl alcohol, nonyl alcohol, decyl alcohol, and undecyl alcohol and combinations thereof, wherein the ester provides a perception of a light skinfeel to a user.

12. The method of claim 11, wherein the at least one component is chosen from an anionic surfactant, a zwitterionic surfactant, a cationic surfactant, a non-ionic surfactant, a lipid, an alcohol, a wax, a pigment, a vitamin, a fragrance, a bleaching agent, an antibacterial agent, an anti-inflammatory agent, an antimycotic agent, a thickener, a starch, chitosan, a polymeric material, a cellulosic material, glycerin, a protein, an amino acid, a keratin fiber, a fatty acid, a siloxane, an abrasive, an exfoliant, lanolin an anticaking agent, an antioxidant agent, a binder, a biological additive, a buffering agent, a bulking agent, a chelating agent, a chemical additive, a denaturant, an external analgesic, a film former, an humectant, an opacifying agent, a pH adjuster, a preservative, a propellant, a reducing agent, a sunscreen agent, a skin darkening agent, an essential oil, and a skin sensate.

13. The method of claim 11, further comprising admixing the ester with at least one vegetable oil prior to combination with the at least one component.

14. The method of claim 11, wherein the at least one vegetable oil is chosen from almond oil, castor oil, coconut oil, corn (maize) oil, cottonseed oil, canola oil, flax seed oil, hempseed oil, nut oil, olive oil, palm oil, peanut oil, safflower oil, sesame oil, soybean oil, sunflower oil, jojoba oil and combinations thereof.

15. The method of claim 11, wherein the undecylenic acid is derived from castor oil.

16. The method of claim 11, wherein the vegetable-derived fatty alcohol is heptyl alcohol.

17. The method of claim 11, wherein the esterification product is heptyl undecylenate.

18. The method of claim 11, wherein the ester comprises a compound represented by Formula (I): 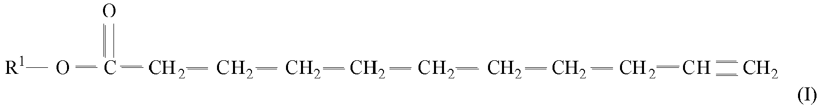 wherein R¹ is chosen from an alkyl group.

19. The method of claim 18, wherein R¹ is chosen from an alkyl group having six to twelve carbon atoms.

20. The method of claim 18, wherein R¹ is chosen from an alkyl group having seven, eight, nine, ten and eleven carbon atoms.

## Patentansprüche

1. Verwendung eines natürlichen Esters zum topischen Auftragen auf die Haut, das Haar und/oder die Nägel, **dadurch gekennzeichnet**, das er das Veresterungsprodukt (1) einer undecylenischen Säure, die aus einer natürlichen Quelle abgeleitet ist, und (2) eines von Pflanzen abgeleiteten Fettalkohols umfasst ausgewählt aus der Gruppe bestehend aus Hexylalkohol, Heptylalkohol, Caprylylalkohol, Nonylalkohol, Decylalkohol und Undecylalkohol und Kombinationen davon, wobei der Ester einem Benutzer eine Empfindung eines leichten Hautgefühls verleiht.

2. Verwendung nach Anspruch 1, wobei die undecylenische Säure von Rizinusöl abgeleitet ist.

3. Verwendung nach Anspruch 2, wobei der von Pflanzen abgeleitete Fettalkohol Heptylalkohol ist.

4. Verwendung nach Anspruch 1, wobei das Veresterungsprodukt Heptylundecylenat ist.

5. Verwendung nach Anspruch 1, wobei das Veresterungsprodukt ein Ester ist, der durch die Formel (I) dargestellt ist: 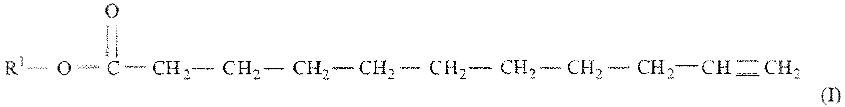 wobei R¹ von einer Alkylgruppe ausgewählt wird.

6. Verwendung nach Anspruch 5, wobei R¹ von einer Alkylgruppe ausgewählt wird, die sechs bis zwölf Kohlenstoffatome aufweist.

7. Verwendung nach Anspruch 5, wobei R¹ von einer Alkylgruppe ausgewählt wird, die sieben, acht, neun, zehn oder elf Kohlenstoffatome aufweist.

8. Verwendung nach Anspruch 5, wobei ein oder mehrere Kohlenstoffatome(e) unabhängig substituiert ist/sind.

9. Verwendung nach Anspruch 1, wobei die Viskosität des Fluids 2 bis 10 Centistoke bei 25 °C beträgt.

10. Verwendung nach Anspruch 1, wobei der Ausbreitungswert des Fluids 6 bis 8 (1 Minute, cm²) beträgt.

11. Verfahren zur Herstellung einer natürlichen Körperpflegezusammensetzung zum topischen Auftragen auf die Haut, das Haar und/oder die Nägel, **dadurch gekennzeichnet, dass** es das Kombinieren eines Esters und mindestens einer Komponente einer Körperpflegezusammensetzung umfasst, wobei der Ester das Veresterungsprodukt (1) einer undecylenischen Säure, die aus einer natürlichen Quelle abgeleitet ist, und (2) eines von Pflanzen abgeleiteten Fettalkohols umfasst ausgewählt aus der Gruppe bestehend aus Hexylalkohol, Heptylalkohol, Caprylylalkohol, Nonylalkohol, Decylalkohol und Undecylalkohol und Kombinationen davon, wobei der Ester einem Benutzer eine Empfindung eines leichten Hautgefühls verleiht.

12. Verfahren nach Anspruch 11, wobei die mindestens eine Komponente unter einem anionischen Tensid, einem zwitterionischen Tensid, einem kationischen Tensid, einem nichtionischen Tensid, einem Lipid, einem Alkohol, einem Wachs, einem Pigment, einem Vitamin, einem Duftstoff, einem Bleichmittel, einem antibakteriellen Mittel, einem entzündungshemmenden Mittel, einem Antimykotikum, einem Verdickungsmittel, einer Stärke, Chitosan, einem polymeren Material, einem cellulosischen Material, Glycerin, einem Protein, einer Aminosäure, einer Keratinfaser, einer Fettsäure, einem Siloxan, einem Abrasivstoff, einem Körperpeeling, Lanolin, einem Antiagglomerationsmittel, einem Antioxidationsmittel, einem Bindemittel, einem biologischen Zusatzmittel, einem Puffermittel, einem Füllstoff, einem Chelatbildner, einem chemischen Zusatzmittel, einem Denaturierungsmittel, ein einem extern anwendbaren Schmerzmittel, einem Filmbildungsmittel, einem Feuchthaltemittel, einem Trübungsmittel, einem pH-Wert-Einstellmittel, einem Konservierungsmittel, einem Treibmittel, einem Reduktionsmittel, einem Sonnenschutzmittel, einem Hauptbräunungsmittel, einem ätherischen Öl und einem Hautsensat ausgewählt wird.

13. Verfahren nach Anspruch 11, ferner das Mischen des Esters mit mindestens einem Pflanzenöl vor der Kombination mit der mindestens einen Komponente umfassend.

14. Verfahren nach Anspruch 11, wobei das mindestens eine Pflanzenöl unter Mandelöl, Rizinusöl, Kokosnussöl, Maisöl, Baumwollsamenöl, Canolaöl, Flachssamenöl, Hanfsamenöl, Nussöl, Olivenöl, Palmöl, Erdnussöl, Safloröl, Sesamöl, Sojabohnenöl, Sonnenblumenöl, Jojobaöl, und Kombinationen davon ausgewählt wird.

15. Verfahren nach Anspruch 11, wobei die undecylenische Säure von Rizinusöl abgeleitet ist.

16. Verfahren nach Anspruch 11, wobei der von Pflanzen abgeleitete Fettalkohol Heptylalkohol ist.

17. Verfahren nach Anspruch 11, wobei das Veresterungsprodukt Heptylundecylenat ist.

18. Verfahren nach Anspruch 11, wobei der Ester eine Verbindung umfasst, die durch die Formel (I) dargestellt ist: 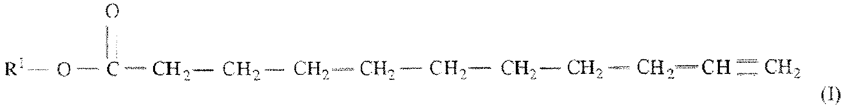 wobei R¹ von einer Alkylgruppe ausgewählt wird.

19. Verfahren nach Anspruch 18, wobei R¹ von einer Alkylgruppe ausgewählt wird, die sechs bis zwölf Kohlenstoffatome aufweist.

20. Verfahren nach Anspruch 18, wobei R¹ von einer Alkylgruppe ausgewählt wird, die sieben, acht, neun, zehn und elf Kohlenstoffatome aufweist

## Revendications

1. Utilisation d'un ester destiné à être appliqué par voie topique sur la peau, les cheveux et/ou les ongles, **caractérisé en ce qu'**il comprend le produit d'estérification (1) d'un acide undécylénique dérivé d'une source naturelle et (2) un alcool gras dérivé d'une plante, choisi dans le groupe constitué d'alcool héxylique, alcool heptylique, alcool caprylique, alcool nonylique, alcool décylique, alcool undécyléniique et leurs combinaisons, dans lequel l'ester permet à un usager d'avoir une perception d'un contact léger sur la peau.

2. Utilisation selon la revendication 1, dans laquelle l'acide undécylénique est dérivé de l'huile de ricin.

3. Utilisation selon la revendication 3, dans laquelle l'alcool gras dérivé d'une plante est alcool heptylique.

4. Utilisation selon la revendication 1, dans laquelle le produit d'estérification est undécylénate d'heptyle.

5. Utilisation selon la revendication 1, dans laquelle ledit produit d'estérification est un ester représenté par la formule (I): 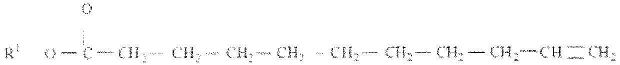 dans laquelle R¹ est choisi parmi des groupes alkyles.

6. Utilisation selon la revendication 5, dans laquelle R¹ est choisi parmi des groupes alkyles ayant six à douze atomes de carbone.

7. Utilisation selon la revendication 5, dans laquelle R¹ est choisi parmi des groupes alkyles ayant sept, huit, neuf, dix ou onze atomes de carbone.

8. Utilisation selon la revendication 5, dans laquelle un ou plusieurs atomes de carbone ou plus(s) est substituée indépendamment.

9. Utilisation selon la revendication 1, dans lequel la viscosité du fluide est de 2 à 10 centistokes à 25 °C.

10. Utilisation selon la revendication 1, dans lequel la vitesse d'étalement du fluide est de 6 à 8 (1 minute, cm²).

11. Procédé de préparation d'une composition naturelle pour les soins personnels destinée à être appliquée par voie topique sur la peau, les cheveux et/ou les ongles, **caractérisé en ce qu'**il comprend l'étame consistant à combiner un ester et au moins une composante pour les soins personnels, dans lequel l'ester comprend le produit d'estérification (1) d'un acide undécylénique dérivé d'une source naturelle et (2) un alcool gras dérivé d'une plante, choisi dans le groupe constitué d'alcool héxylique, alcool heptylique, alcool caprylique, alcool nonylique, alcool décylique, alcool undécyléniique et leurs combinaisons, dans lequel l'ester permet à un usager d'avoir une perception d'un contact léger sur la peau.

12. Procédé selon la revendication 11, dans lequel l'au moins une composante est choisie parmi un tensioactif anionique, un tensioactif zwitterionique, un tensioactif cationique, un tensioatif non ionique, un lipide, un alcool, une cire, un pigment, une vitamine, un parfum, un agent de blanchiment, un agent antibactérien, un agent anti-inflammatoire, un agent antimycosique, un épaississant, un amidon, chitosane, un matériau polymérique, un matériau cellulosique, glycérine, une protéine, un acide aminé, une fibre de kératine, un acide gras, un siloxane, un abrasif, un exfoliant, lanoline, un agent anti - agglomérant, un antioxydant, un liant, un additif biologique, un agent tampon, un agent gonflant, un agent chélateur, un additif chimique, un dénaturant, un analgésique externe, un filmogène, un humectant, un agent opacifiant, un régulateur de pH, un agent de conservation, un agent propulseur, un agent réducteur, un agent de protection solaire, un agent bronzant, une huile essentielle et un agent sensoriel de la peau.

13. Procédé selon la revendication 11, comprenant également l'étape de mélanger l'ester avec au moins une huile végétale avant la combinaison avec l'au moins une composante.

14. Procédé selon la revendication 11, dans lequel l'huile végétale est choisie parmi l'huile d'amande, l'huile de ricin, l'huile de noix de coco, l'huile de maïs, l'huile de coton, l'huile de colza, l'huile de lin, l'huile de chanvre, l'huile de noix, l'huile d'olive, l'huile de palme, l'huile d'arachide, l'huile de carthame, l'huile de sésame, l'huile de soja, l'huile de tournesol, l'huile de jojoba et leurs combinaisons.

15. Procédé selon la revendication 11, dans laquelle l'acide undécylénique est dérivé de l'huile de ricin.

16. Procédé selon la revendication 11, dans laquelle l'alcool gras dérivé d'une plante est alcool heptylique.

17. Procédé selon la revendication 11, dans laquelle le produit d'estérification est undécylénate d'heptyle.

18. Procédé selon la revendication 11, dans lequel l'ester comprend un composé représenté par la formule (I): 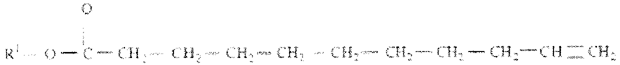 dans laquelle R¹ est choisi parmi des groupes alkyles.

19. Procédé selon la revendication 18, dans laquelle R¹ est choisi parmi des groupes alkyles ayant six à douze atomes de carbone.

20. Procédé selon la revendication 18, dans laquelle R¹ est choisi parmi des groupes alkyles ayant sept, huit, neuf, dix et onze atomes de carbone.
